# EUROPÄISCHE PATENTSCHRIFT

(11) **EP 1 092 700 B1**
(45) Veröffentlichungstag und Bekanntmachung des Hinweises auf die Patenterteilung: **22.10.2003**
(21) Anmeldenummer: 00121161.4
(22) Anmeldetag: 29.09.2000
(51) Int. Cl.: C07C 45/51, C07C 49/203

(54) **Verfahren zur Herstellung von ungesättigten 4,5-Allenketonen, 3,5 Dienketonen und den entsprechenden gesättigten Ketonen**
Process for the preparation of unsaturated 4,5-allene ketones, 3,5-dienketones and the corresponding saturated ketones
Procédé de préparation de cétones 4,5 alléniques insaturées, de cétones 3,5 diéniques et les cétones saturées correspondantes

(30) Priorität: 15.10.1999 DE 19949796
(43) Veröffentlichungstag der Anmeldung: 18.04.2001
(73) Patentinhaber: Degussa AG, 40474 Düsseldorf (DE)
(72) Erfinder: Nongyuan, Shi, Dr., 63457 Hanau (DE); Hübner, Frank, Dr., D-64372 Ober-Ramstadt (DE); Drapal, Bernd, 63755 Alzenau (DE); Peter, Rainer, 63829 Krombach (DE); Krill, Steffen, Dr., 67346 Speyer (DE); Huthmacher, Klaus, Dr., 63584 Gelnhausen (DE); Weckbecker, Christoph, Dr., 63584 Gründau-Lieblos (DE)

(56) Entgegenhaltungen:
- DE-B- 1 215 694
- DE-B- 1 230 783
- US-A- 3 029 287

## Beschreibung

Die Erfindung betrifft ein neues Verfahren zur Herstellung von 4,5-Allenketonen nach der Saucy-Marbet-Reaktion durch Umsetzung tertiärer Propargylalkohole mit Alkenylethern in Gegenwart von bestimmten Sulfonsäuren oder Sulfonsäuresalzen. Dadurch entstehende 4,5-Allenketone können in an sich bekannter Weise durch eine anschließende Isomerisierung zu 3,5-Dienketonen oder durch eine anschließende Hydrierung zu gesättigten Ketonen umgewandelt werden. Eine Reihe von 4,5-Allenketonen, 3,5-Dienonen bzw. die entsprechenden gesättigten Ketone sind wertvolle Zwischenprodukte für die Herstellung von Vitamin E, A, K₁ und Carotinoiden.

In der Patentschrift US 3,029,287 bzw. in der Veröffentlichung von R. Marbet und G. Saucy, Helv. Chim. Acta (1967), 50, 1158-1167 ist ein Verfahren zur Herstellung von 4,5-Allenketonen durch Umsetzung von Propargylalkoholen mit Alkenylethern in Gegenwart eines sauren Katalysators beschrieben. Als besonders geeigneter saurer Katalysator wird hierin p-Toluolsulfonsäure genannt. Die Reaktionszeiten, die zum Erreichen eines vollständigen Umsatzes des tertiären Propargylalkohols benötigt werden, betragen jedoch über 15 Stunden. Diese langen Reaktionszeiten verursachen in einem technischen Verfahren große Reaktionsvolumina und hohe Investionskosten.

Nach der EP 0 902 001 A1 erfolgt die Herstellung von 4,5-Allenketonen durch Umsetzung von Propargylalkoholen mit Alkenylethern bei erhöhter Temperatur und Druck in Anwesenheit von KHSO₄ unter Bildung von Ketalen. Das erhaltene Ketal kann wieder in den Alkenylether zurückgeführt werden. Für diese Reaktion mit Propin oder Allen oder deren Mischungen in der Gasphase bei erhöhter Temperatur ist ein Heterogenkatalysator bestehend aus einem röntgenamorphen Zinksilikat notwendig, der die Wirtschaftlichkeit des Verfahrens einschränkt.

Es bestand daher die Aufgabe, die Raum-Zeit Ausbeute bei der Reaktion zwischen tertiären Propargylalkoholen mit Alkenylethern zu verbessern und gute Ausbeuten zu erzielen.

Es wurde nun gefunden, daß aliphatische Sulfonsäuren und Sulfonsäuresalze die Umsetzung von tertiären Propargylalkoholen mit Alkenylethern sehr selektiv katalysieren, wobei die Reaktionszeiten zum Erreichen guter Ausbeuten verkürzt sind.

Gegenstand der Erfindung ist ein Verfahren zur Herstellung von 4,5-Allenketonen der allgemeinen Formel I 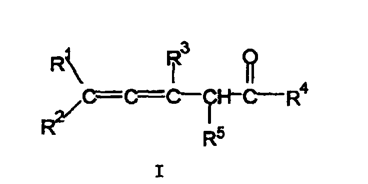 in der
- R¹ und R²: für einen gesättigten oder ungesättigten, verzweigten oder unverzweigten C₁ bis C₂₀-Alkylrest, einen Arylrest oder einen Alkylarylrest stehen, wobei die Reste R¹ und R² auch zusammen einen 5- oder 6-gliedrigen Ring bilden können,
- R³ und R⁵: für Wasserstoff oder einen C₁ bis C₄-Alkylrest stehen, und
- R⁴: für einen C₁ bis C₄ Alkylrest steht,
durch Umsetzen eines tertiären Propargylalkohols der allgemeinen Formel II 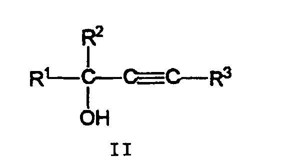 in der R¹, R² und R³ die oben angegebene Bedeutung haben, mit einem Alkenylalkylether der allgemeinen Formel III 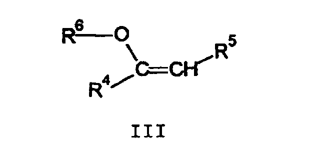 in der R⁴, R⁵ die oben angegebene Bedeutung haben, R⁶ für einen C₁ bis C₄ Alkylrest steht
(oder einem Ketal der allgemeinen Form IV) 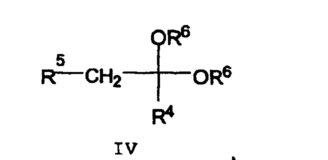 in der
- R⁴, R⁵ und R⁶: die oben angegebene Bedeutung haben, bei Temperaturen von 40 bis 200°C ohne Lösungsmittel oder in einem inerten organischen Lösungsmittel bei Atmosphäre oder unter Druck bis zu 100 bar in Gegenwart einer aliphatischen oder alicyclischen Sulfonsäure der allgemeinen Formel V

R⁷SO₃H V
in der
- R⁷: für Halogen, einen verzweigten oder unverzweigten, gegebenenfalls Halogen-substituierten Alkylrest mit 1 bis 20 C-Atomen oder einen Cycloalkylrest steht,
oder in Gegenwart eines Sulfonsäuresalzes der allgemeinen Formel VI

R⁸SO₃M VI

in der
- R⁸=R⁷, und M: für ein Kation einer organischen oder anorganischen Base steht.

Bevorzugte Ausgangsmaterialien der Formel II sind vor allem tertiäre Propargylalkohole, wobei vorzugsweise
- R¹: für einen gesättigten oder ungesättigten, verzweigten oder unverzweigten C₁ bis C₂₀-Alkylrest, einen Arylrest, oder einen Arylalkylrest steht,
- R²: für einen C₁ bis C₄ Alkylrest, insbesondere einen Methylrest, steht.

Als geeignete Propargylalkohole seien beispielsweise genannt:
3-Methyl-1-butyn-3-ol,
3,7-Dimethyl-6-octen-1-yn-3-ol (Dehydrolinalool)
3,7-Dimethyl-5-octen-1-yn-3-ol
3,7-Dimethyl-4-octen-1-yn-3-ol
3,7-Dimethyl-1-octyn-3-ol (Hydrodehydrolinalool)
3,7,11-Trimethyl-6,10-dodecadien-1-yn-3-ol (Dehydronerolidol)
3,7,11-Trimethyl-6-dodecen-1-yn-3-ol
3,7,11-Trimethyl-1-dodecyn-3-ol (Hydrodehydronerolidol)
1-Ethynyl-1-cyclohexanol,
1-Ethynyl-2,2,6-Trimethyl-1-cyclohexanol.

Als Alkenylalkylether der allgemeinen Formel III kommen vorzugsweise Verbindungen in Frage, bei deren
- R⁴: für Wasserstoff oder einen Methylrest steht,
- R⁵: für Wasserstoff oder einen Methylrest steht,
- R⁶: für einen Methyl-, Ethyl-, Propyl-, Isopropyl-, Isobutyl- oder tert. Butylrest steht.

Als geeignete Alkenylether seien beispielsweise genannt: Isopropenylmethylether, Isopropenylethylether, Isopropenylpropyl-ether, Isopropenylbutylether, Isopropenylisobutylether, Isobutylisopropenylether, Diisopropenylether, Isopropenylphenylether, 2-Methoxy-1-buten, 2-Ethoxy-1-buten, 2-Propoxy-1-buten, 3-Butoxy-1-buten, 2-Methoxy-2-buten, 2-Ethoxy-2-buten, 2-Methoxy-1-penten, 2-Ethoxy-1-penten, 2-Methoxy-2-penten, 2-Ethoxy-2-penten, 3-Methoxy-3-penten, 3-Ethoxy-2-penten, insbesondere Isopropenylmethylether, Isopropenylethylether und Isopropenylisopropylether.

Als geeignete aliphatische Sulfonsäuren seien beispielsweise genannt:

Methansulfonsäure, Ethansulfonsäure, Propansulfonsäure, Butansulfonsäure, Pentansulfonsäure, Hexansulfonsäure, halogensubstituierte Methansulfonsäure, Fluorsulfonsäure und Cyclohexansulfonsäure, insbesondere Methan- und Ethansulfonsäure.

Als geeignete Sulfonsäuresalze seien beispielsweise genannt:

Pyridinium-p-toloulsulfonat, Tetramethylammonium-ptoluolsulfonat, Pyridiniummethansulfonat,

Pyridiniumethansulfonat, insbesondere Pyridinium-p-toloulsulfonat.

Die Sulfonsäuren und die genannten Sulfonsäuresalze können entweder direkt eingesetzt werden oder in situ über die entsprechenden Sulfonsäurechloride oder -anhydride in bekannter Weise hergestellt werden.

Die Reaktion erfolgt zweckmäßigerweise bei Temperaturen zwischen etwa 40°C und 200°C, vorzugsweise zwischen etwa 50°C und 120°C.

Die Reaktion kann drucklos aber auch unter Druck durchgeführt werden. Bei einer Druckreaktion erfolgt die Reaktion in einem Druckbereich von 1 bis 100 bar, vorzugsweise von 1 bis 20 bar. Das Verfahren kann auch wie folgt durchgeführt werden: In einer 1. Stufe bei Atmosphäre und anschließend die 2. Stufe unter Überdruck.

Das Molverhältnis zwischen dem tertiären Alkohol der allgemeinen Formel II und dem Alkenylalkylether der allgemeinen Formel III liegt beim erfindungsgemäßen Verfahren im allgemeinen zwischen 1:2 bis 1:10, vorzugsweise zwischen 1:2 bis 1:3. In einer Reaktion ohne Lösungsmittel dient der überschüssige Alkenylalkylether als Lösungsmittel und kann nach Beendigung der Reaktion destillativ zurückgewonnen werden.

Die Saucy-Marbet-Reaktion kann mit oder ohne Lösungsmittel durchgeführt werden. Als geeignete Lösungsmittel können im Rahmen der vorliegenden Erfindung Kohlenwasserstoffe, z. B. Hexan, Heptan, Octan, Toluol, und Xylol oder Ketone und Ether z. B. Isobutylmethylketon, Diethylketon, Isophoron oder Dimethoxypropan eingesetzt werden.

Zur Durchführung des Verfahrens geht man im allgemeinen so vor, daß man entweder ein Gemisch aus dem tertiären Propargylalkohol und dem Alkenylalkylether im Reaktionsgefäß vorlegt und den Katalysator in Form eines Feststoffes, einer Schmelze oder insbesondere gelöst in einem geeigneten Lösemittel entweder kontinuierlich oder portionsweise zugibt. Danach wird das Gemisch auf die Reaktionstemperatur erwärmt. Man kann den sauren Katalysator mit den Reaktanden vorlegen oder während der Reaktion portionsweise zudosieren.

Die Umsetzung kann diskontinuierlich, aber auch kontinuierlich durchgeführt werden.

Das entstehende Produkt 4,5-Allenketon kann entweder direkt isoliert werden oder durch basische Isomerisierung in an sich bekannter Weise (siehe US 3.029.287) zu 3,5-Dienon der allgemeinen Form 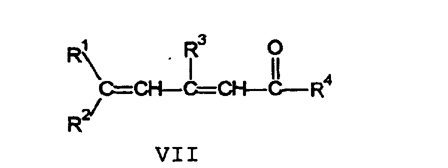 in der R¹, R², R³ und R⁴ oben angegebene Bedeutung haben,
umgewandelt werden; letztere können als wichtige Zwischenstufen für Vitamin-A, E, K₁ und Carotinoide verwendet werden.

Die entstehenden 4,5-Allenketone können aber auch in an sich bekannter Weise (R. Marbet und G. Saucy, Helv. Chim. Acta (1967),50, 1158-1167) zu gesättigten Ketonen
der allgemeinen Form VIII 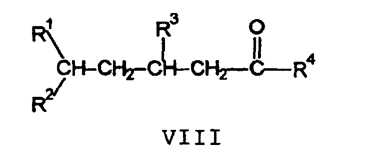 in der R¹, R², R³ und R⁴ oben angegebene Bedeutung haben,
hydriert werden. Die gesättigten Ketone können wichtige Zwischenprodukte für die Vitamin-E-Synthese sein.

Die folgenden Beispiele erläutern die Erfindung. Die eingesetzten Propargylalkohole sind in der US 3.029.287 beschrieben.

### Beispiel 1

In einem 250 ml Kolben wurden 30,5 g 3,7-Dimethyl-1-octin-6-en-3-ol, 28,9 g Isopropenylmethylether, 60 ml n-Heptan vorgelegt, anschließend wurden 7,7 mg Methansulfonsäure zugegeben. Die Reaktionsmischung wurde 7,5 Stunden unter Rückfluß gerührt. Während dieser Zeit wurden 14,4 g Isopropenylmethylether und 11,6 mg Methansulfonsäure in zwei Portionen ergänzt. Nach dem Abkühlen wurde mit 2 ml methanolischer NaOAc-Lösung (10g/l) neutralisiert und eingedampft. Das erhaltene rohe Allenketon wurde in 2-Propanol mit 10%-Pd/A-Kohle zu Tetrahydrogeranylaceton hydriert. Die Gesamtausbeute von Tetrahydrogeranylaceton betrug 90%.

### Beispiel 2

In einem 250 ml Kolben wurden 30,5 g 3,7-Dimethyl-1-octin-6-en-3-ol, 36 g Isopropenylmethylether und 60 ml n-Heptan vorgelegt, anschließend wurden 22 mg Ethansulfonsäure zugegeben. Die Reaktionsmischung wurde unter Rückfluß 7 Stunden gerührt. Während dieser Zeit wurden 7,3 g Isopropenyl-methylether und 4,4 mg Ethansulfonsäure ergänzt. Nach Abkühlen auf Raumtemperatur wurde mit 2 ml methanolischer NaOAc-Lösung (10g/l) neutralisiert und eingedampft. Das erhaltene rohe Allenketon wurde in 2-Propanol mit 10%-Pd/A-Kohle zu Tetrahydrogeranylaceton hydriert. Man erhielt Tetrahydrogeranylaceton in einer Ausbeute von 88%.

### Beispiel 3

In einem 250 ml Kolben wurden 30,5 g 3,7-Dimethyl-1-octin-6-en-3-ol, 28,9 g Isopropenylmethylether und 60 ml n-Heptan vorgelegt, anschließend wurden 25 mg Pyridinium-toluol-4-sulfonat zugegeben. Die Reaktionsmischung wurde 7,5 Stunden unter Rückfluß gerührt. Während dieser Zeit wurden 14,4 g Isopropenylmethylether und 50 mg Pyridinium-toluol-4-sulfonat in zwei Portionen ergänzt. Die Reaktionsmischung wurde nach Abkühlen mit 2,5 ml methanolischer NaOAc-Lösung (10g/l) neutralisiert und eingedampft. Das erhaltene rohe Allenketon wurde in 2-Propanol mit 10%-Pd/A-Kohle zu Tetrahydrogeranylaceton hydriert. Die Gesamtausbeute von Tetrahydrogeranylaceton betrug 90%.

### Beispiel 4

In einem 500 ml Kolben wurden 60,9 g 3,7-Dimethyl-6-octen-1-in-3-ol, 46,2 g Isopropenylmethylether und 160 mL n-Heptan unter Stickstoff vorgelegt. Unter Rühren wurden 23 mg Methansulfonsäure zugegeben. Die Reaktionsmischung wurde unter Rückfluß 6 Stunden gerührt. Während dieser Zeit wurde 56,3 g Isopropenylmethylether in zwei Portionen, 44,3 mg Methansulfonsäure in 3 Portionen zugegeben. Es wurde auf Raumtemperatur gekühlt und durch Zusatz von 5,75 mL einer methanolischen NaOAc-Lösung (10mg/mL) neutralisiert. Das Reaktionsgemisch wurde eingedampft. Zur Umlagerung wurde das erhaltene Rohprodukt von Allenketon unter kräftigem Rühren bei 0 bis 10°C in ein Gemisch aus 40 mL Methanol und 0,5 mL 30 %-iger Natronlauge zugegeben. 40 mL n-Heptan wurden noch dazugegeben. Nach Zusatz von 0,3 mL Eisessig wurde die Mischung auf Raumtemperatur erwärmt und eingedampft. Der Rückstand wurde mit 150 mL n-Hexan aufgenommen und mit Wasser gewaschen. Die organische Phase wurde mit Magnesiumsulfat getrocknet und eingedampft. Nach Destillation unter Vakuum erhielt man 72,8 g Pseudoionon, entsprechend einer Ausbeute von 92%.

### Beispiel 5

Der Versuch wurde analog des Beispiels 4 durchgeführt, wobei anstelle von Methansulfonsäure 176 mg Pyridiniumtoluol-4-sulfonat als Katalysator eingesetzt wurden. Nach Umlagerung, Aufarbeitung und Destillation erhielt man 71,1 g Pseudoionon, entsprechend eine Ausbeute von 92%.

### Beispiel 6

In einem 250 ml Kolben wurden 30,9g 3,7-Dimethyl-1-octin-3-ol, 28,9 g Isopropenylmethylether und 60 ml n-Heptan vorgelegt, anschließend wurden 7,7 mg Methansulfonsäure zugegeben. Die Reaktionsmischung wurde unter Rückfluß 7,7 Stunden gerührt. Während dieser Zeit wurden 14,4 g Isopropenyl-methylether und 7,7 mg Methansulfonsäure ergänzt. Die Mischung wurde abgekühlt und mit 1,5 ml methanolischer NaOAc-Lösung (10g/l) neutralisiert und eingedampft. Das Rohprodukt Allenketon wurde in 2-Propanol mit 10%-Pd/A-Kohle zu Tetrahydrogeranylaceton hydriert. Die Gesamtausbeute von Tetrahydrogeranylaceton betrug 89%.

### Beispiel 7

Der Versuch wurde analog des Beispiels 6 durchgeführt, wobei anstelle von Methansulfonsäure 75 mg Pyridiniumtoluol-4-sulfonat als Katalysator eingesetzt wurden. Nach Hydrierung und Aufarbeitung erhielt man Tetrahydrogeranylaceton in einer Gesamtausbeute von 90%.

### Beispiel 8

In einem 250 ml Kolben wurden 30,9 g 3,7-Dimethyl-1-octin-3-ol, 28,9 g Isopropenylmethylether, 60 ml n-Heptan vorgelegt, anschließend wurden 25 mg Pyridinium-toluol-4-sulfonat zugegeben. Die Reaktionsmischung wurde 7 Stunden unter Rückfluß gerührt. Während dieser Zeit wurden 14,4 g Isopropenylmethylether und 25 mg Pyridinium-toluol-4-sulfonat in zwei Portionen zugegeben. Nach Beendigung der Reaktion wurde die Lösung eingedampft. Der Rückstand wurde unter Vakuum destilliert. Man erhielt 31,9 g 6,10-Dimethyl-4,5-undecadien-2-on, dies entspricht eine Ausbeute von 82%.

### Beispiel 9

In einem 500 ml Kolben wurden 21,1g 2-Methyl-3-butin-2-ol, 54,1 g Isopropenylmethylether, 0,05 g Hydrochinon und 100 mL n-Heptan vorgelegt. Unter Rühren wurden 20,7 mg Methansulfonsäure zugegeben. Die Reaktionsmischung wurde 6,5 Stunden unter Rückfluß gerührt. Nach Abkühlen wurde mit 2 ml methanolischer NaOAc-Lösung (10g/l) neutralisiert und eingedampft. Nach Destillation in Vakuum erhielt man 27,5 g 6-Methyl-4,5-heptadien-2-on, entsprechend einer Ausbeute von 89% .

### Beispiel 10

Der Versuch wurde analog des Beispiels 9 durchgeführt, wobei anstelle von Methansulfonsäure 56,6 mg Pyridiniumtoluol-4-sulfonat als Katalysator eingesetzt wurden. Die Ausbeute von 6-Methyl-4,5-heptadien-2-on betrug 91%.

### Beispiel 11

In einem 500 ml Kolben wurden 56,1g 3,7,11-Trimethyl-1-dodecin-3-ol, 27,0 g Isopropenylmethylether und 100 mL n-Heptan vorgelegt. Unter Rühren wurden 14,5 mg Methansulfonsäure zugegeben. Die Reaktionsmischung wurde unter Rückfluß 7,5 Stunden gerührt. Während dieser Zeit wurde 36,5 g Isopropenylmethylether und 21,6 mg Methansulfonsäure in jeweiligen zwei Portionen zugegeben. Es wurde auf Raumtemperatur gekühlt und mit 3 ml NaOAc-Lösung in Methanol (10g/l) neutralisiert und eingedampft. Das Rohprodukt wurde in Isopropanol mit 10%-Pd/C hydriert. Man erhielt Phyton in einer Ausbeute von 91 %.

### Beispiel 12

Der Versuch wurde analog des Beispiels 11 durchgeführt, wobei anstelle von Methansulfonsäure 94,2 mg Pyridiniumtoluol-4-sulfonat in 3 Portionen als Katalysator eingesetzt wurden. Nach Aufarbeitung und Hydrierung erhielt man Phyton in einer Ausbeute von 91%.

### Beispiel 13

Der Versuch wurde analog des Beispiels 11 durchgeführt, wobei anstelle von Methansulfonsäure 41,3 mg Ethansulfonsäure in 3 Portionen als Katalysator zugesetzt wurden. Nach Aufarbeitung und Hydrierung erhielt man Phyton in einer Ausbeute von 91%.

### Beispiel 14

In einem 300 ml V4A-Stahlautoklaven mit Temperaturfühler, Probenahme und Druckanzeige wurden 44,9 g 3,7,11-Trimethyl-1-dodecin-3-ol und 50,5 g Isopropenylmethylether vorgelegt. Unter Rühren wurden 19 mg Methansulfonsäure zugesetzt. Der Autoklav wurde mit Stickstoff auf 2 bar aufgestockt. Es wurde 8 Stunden bei 90 bis 95°C gerührt. Nach Abkühlen wurde die Reaktionsmischung mit 1,7 ml methanolischer NaOAc-Lösung (10g/l) neutralisiert. Nach Eindampfen wurde das Rohprodukt in 2-Propanol mit 10%-Pd/A-Kohle hydriert. Man erhielt Phyton in einer Ausbeute von 87%.

### Beispiel 15

In einem 500 ml Kolben wurden 44,1g 3,7,11-Trimethyl-6,10-dodecadien-1-in-3-ol, 50,5 g Isopropenylmethylether und 80 mL n-Heptan vorgelegt. Unter Rühren wurden 29 mg Methansulfonsäure zugegeben. Die Reaktionsmischung wurde unter Rückfluss 6 Stunden gerührt. Nach Reaktionsende wurde auf Raumtemperatur gekühlt, mit 2,5 ml methanolischer NaOAc-Lösung (10g/l) neutralisiert und eingedampft. Der Rückstand wurde in 2-Propanol mit 10%-Pd/A-Kohle hydriert.

Man erhielt Phyton in einer Ausbeute von 76%.

### Beispiel 16

Der Versuch wurde analog des Beispiels 15 durchgeführt, wobei anstelle von Methansulfonsäure 33 mg Ethansulfonsäure als Katalysator eingesetzt wurden. Man erhielt Phyton in einer Ausbeute von 73%.

### Beispiel 17

In einem 500 ml Kolben wurden 25,3 g 2-Methyl-3-butin-2-ol, 65,0 g Isopropenylmethylether, 0,066 g Hydrochinon sowie 120 ml n-Heptan unter Stickstoff vorgelegt. Unter Rühren wurden 15,0 mg Fluorsulfonsäure zugegeben. Die Mischung wurde zum Sieden erhitzt und 9 h bei 60 bis 77°C unter Rückfluß gerührt. Nach Abkühlen wurde mit 2 ml methanolischer NaOAc-Lösung (10g/l) neutralisiert und eingedampft. Nach Destillation erhielt man 32,7 g 6-Methyl-4,5-heptadien-2-on, dies entspricht einer Ausbeute von 87,8%.

## Patentansprüche

1. Verfahren zur Herstellung von ungesättigten 4,5-Allenketonen, der allgemeinen Formel I 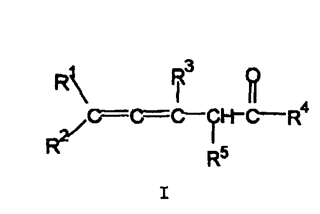 in der
R¹ und R² für einen gesättigten oder ungesättigten, verzweigten oder unverzweigten C₁ bis C₂₀-Alkylrest, einen Arylrest oder einen Alkylarylrest stehen, wobei die Reste R¹ und R² auch zusammen einen 5- oder 6-gliedrigen Ring bilden können,
R³ und R⁵ für Wasserstoff oder einen C₁ bis C₄-Alkylrest stehen und
R⁴ für einen C₁ bis C₄ Alkylrest steht,
durch Umsetzen eines tertiären Propargylalkohols der allgemeinen Formel II 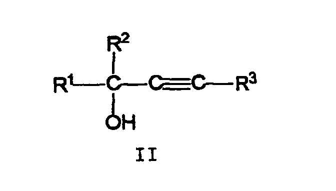 in der R¹, R² und R³ die oben angegebene Bedeutung haben,
mit einem Alkenylalkylether der allgemeinen Formel III 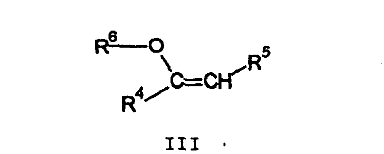 in der R⁴, R⁵ die oben angegebene Bedeutung haben, R⁶ für einen C₁ bis C₄ Alkylrest steht
oder einem Ketal der allgemeinen Formel IV 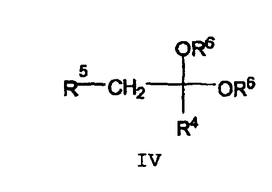 in der
R⁴, R⁵ und R⁶ die oben angegebene Bedeutung haben,
bei Temperaturen von 40 bis 200°C ohne Lösungsmittel oder in einem inerten organischen Lösungsmittel bei Atmosphäre oder unter Druck bis zu 100 bar **dadurch gekennzeichnet, daß** die Reaktion in Gegenwart einer aliphatischen oder alicyclischen Sulfonsäure der allgemeinen Formel V
R⁷SO₃H V
in der
R⁷ für Halogen, einen verzweigten oder unverzweigten, gegebenenfalls Halogensubstituierten Alkylrest mit 1 bis 20 C-Atomen oder einen Cycloalkylrest steht,
oder in Gegenwart eines Sulfonsäuresalzes der allgemeinen Formel VI
R⁸SO₃M VI
in der
R⁸=R⁷ und M für ein Kation einer organischen oder anorganischen Base steht, durchgeführt wird.

2. Verfahren gemäß Anspruch 1, **dadurch gekennzeichnet, daß** die 4,5-Allenketone der allgemeinen Formel I 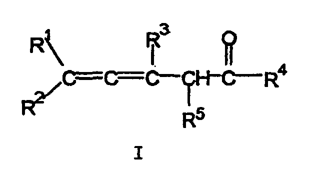
R⁷SO₃H V
R⁸SO₃M VI
durch nachfolgende Isomerisierung oder Hydrierung zu den entsprechenden ungesättigten 3,5-Dienketonen oder gesättigten Ketonen umgesetzt werden.

3. Verfahren nach Anspruch 1, **dadurch gekennzeichnet, daß** die 4,5-Allenketone der allgemeinen Formel I 6,10-Dimethyl-4,5-undecadien-2-on und 6-Methyl-4, 5-heptadien-2-on, 6,10-Dimethyl-4,5,9-undecatrien-2-on, 6,10,14-Trimethyl-4,5,9,13-pentadecatetraen-2-on und 6,10,14-Trimethyl-4,5-pentadecadien-2-on bedeuten.

4. Verfahren nach Anspruch 1, **dadurch gekennzeichnet, daß** die Sulfonsäuren der allgemeinen Formel V Methan-, Ethan-, Propan-, Butan-, Pentan-, Hexan-, halogensubstituierte Methan-, und Cyclohexansulfonsäure bedeuten.

5. Verfahren nach Anspruch 1, **dadurch gekennzeichnet, daß** die Sulfonsäuresalze der allgemeinen Formel VI Pyridinium-p-toluolsulfonat, Tetramethylammonium-ptoluolsulfonat, Pyridiniummethansulfonat, Pyridiniumethansulfonat bedeuten.

6. Verfahren nach Anspruch 1, **dadurch gekennzeichnet, daß** die Herstellung der 4,5-Allenketone der allgemeinen Formel I bei Temperaturen zwischen 50°C und 120°C und bei Arbeiten unter Druck zwischen 1 bis 20 bar erfolgt.

7. Verfahren nach Anspruch 1, **dadurch gekennzeichnet, daß** die Herstellung der 4,5-Allenketone der allgemeinen Formel I in einem organischen Kohlenwasserstoff, Aromaten, Keton oder Äther als Lösungsmittel vorgenommen wird.

## Claims

1. Process for the preparation of unsaturated 4,5-allene ketones, of the general formula I 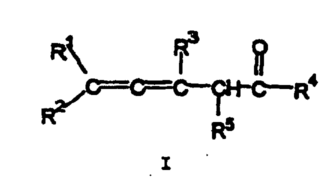 in which
R¹ and R² represent a saturated or unsaturated, branched or unbranched C₁ to C₂₀- alkyl radical, an aryl radical or an alkylaryl radical, it also being possible for the radicals R¹ and R² together to form a 5- or 6-membered ring,
R³ and R⁵ represent hydrogen or a C₁ to C₄-alkyl radical and
R⁴ represents a C₁ to C₄ alkyl radical,
by reacting a tertiary propargyl alcohol of the general formula II 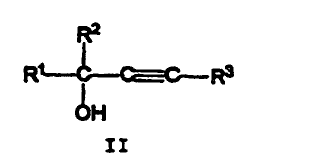 in which R¹, R² and R³ have the abovementioned meaning,
with an alkenyl alkyl ether of the general formula III 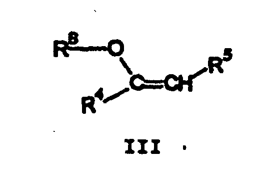 in which R⁴, R⁵ have the abovementioned meaning, R⁶ represents a C₁ to C₄ alkyl radical
or a ketal of the general formula IV 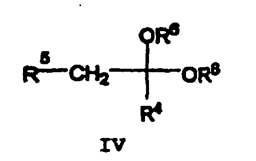 in which
R⁴, R⁵ and R⁶ have the abovementioned meaning
at temperatures of 40 to 200°C without a solvent or in an inert organic solvent in the atmosphere or under a pressure of up to 100 bar, **characterized in that** the reaction is carried out in the presence of an aliphatic or alicyclic sulfonic acid of the general formula V
R⁷SO₃H V
in which
R⁷ represents halogen, a branched or unbranched, optionally halogen-substituted alkyl radical having 1 to 20 C atoms or a cycloalkyl radical,
or in the presence of a sulfonic acid salt of the general formula VI
R⁸SO₃M VI
in which
R⁸=R⁷ and M represents a cation of an organic or inorganic base.

2. Process according to claim 1, **characterized in that** the 4,5-allene ketones of the general formula I 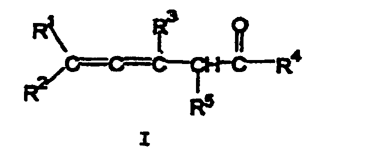 are converted into the corresponding unsaturated 3,5-diene ketones or saturated ketones by subsequent isomerization or hydrogenation.

3. Process according to claim 1, **characterized in that** the 4,5-allene ketones of the general formula I denote 6,10-dimethyl-4,5-undecadien-2-one and 6-methyl-4,5-heptadien-2-one, 6,10-dimethyl-4,5,9-undecatrien-2-one, 6,10,14-trimethyl-4,5,9,13-pentadecatetraen-2-ones and 6,10,14-trimethyl-4,5-pentadecadien-2-one.

4. Process according to claim 1, **characterized in that** the sulfonic acids of the general formula V denote methane-, ethane-, propane-, butane-, pentane-, hexane-, halogen-substituted methane- and cyclohexanesulfonic acid.

5. Process according to claim 1, **characterized in that** the sulfonic acid salts of the general formula VI denote pyridinium p-toluenesulfonate, tetramethylammonium p-toluenesulfonate, pyridinium methanesulfonate, pyridinium ethanesulfonate.

6. Process according to claim 1, **characterized in that** the preparation of the 4,5-allene ketones of the general formula I is carried out at temperatures of between 50°C and 120°C, working under a pressure of between 1 to 20 bar.

7. Process according to claim 1, **characterized in that** the preparation of the 4,5-allene ketones of the general formula I is carried out in an organic hydrocarbon, aromatic, ketone or ether as the solvent.

## Revendications

1. Procédé pour la préparation de 4,5-allènecétones insaturées de formule générale I 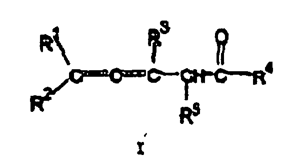 dans laquelle
R¹ et R² représentent un radical alkyle en C₁ à C₂₀ saturé ou insaturé, ramifié ou non ramifié, un racial aryle ou un radical alkylaryle, les radicaux R¹ et R² pouvant également former ensemble un cycle à 5 ou 6 membres,
R³ et R⁵ représentent un atome d'hydrogène ou un radical alkyle en C₁ à C₄,
R⁴ représente un radical alkyle en C₁ à C₄,
par transformation d'un alcool propargylique tertiaire de formule générale II 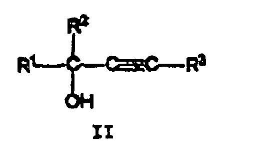 dans laquelle R¹, R² et R³ ont la signification indiquée ci-dessus
avec un alcénylalkyléther de formule générale III 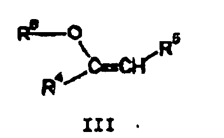 dans laquelle R⁴, R⁵ ont la signification susmentionnée, R⁶ représente un radical alkyle en C₁ à C₄,
ou avec un cétal de formule générale IV 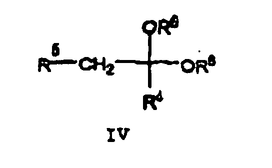 dans laquelle R⁴, R⁵ et R⁶ ont la signification susmentionnée
à des températures de 40 à 200°C, sans solvant ou dans un solvant organique inerte à la pression atmosphérique ou à une pression jusqu'à 100 bars, **caractérisé en ce que** la réaction est réalisée en présence d'un acide sulfonique aliphatique ou alicyclique de formule générale V
R⁷SO₃H V
dans laquelle R⁷ représente un atome d'halogène, un radical alkyle ramifié ou non ramifié, le cas échéant substitué par halogène, comprenant 1 à 20 atomes de carbone ou un radical cycloalkyle
ou en présence d'un sel d'acide sulfonique de formule générale VI
R⁸SO₃M VI
dans laquelle R⁸ = R⁷
et
M représente un cation d'une base organique ou inorganique.

2. Procédé selon la revendication 1, **caractérisé en ce que** les 4,5-allènecétones de formule générale 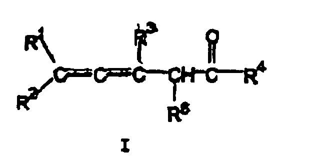 sont transformées par isomérisation ou hydrogénation consécutive en 3,5-diènecétones insaturées ou en cétones saturées correspondantes.

3. Procédé selon la revendication 1, **caractérisé en ce que** les 4,5-allènecétones de formule générale I signifient la 6,10-diméthyl-4,5-undécadién-2-one et la 6-méthyl-4,5-heptadién-2-one, la 6,10-diméthyl-4,5,9-undécatrién-2-one, la 6,10,14-triméthyl-4,5,9,13-pentadécatétraén-2-one et la 6,10,14-triméthyl-4,5-pentadécadién-2-one.

4. Procédé selon la revendication 1, **caractérisé en ce que** les acides sulfoniques de formule générale V signifient l'acide méthanesulfonique, éthanesulfonique, propanesulfonique, butanesulfonique, pentanesulfonique, hexanesulfonique, méthanesulfonique substitué par halogène et cyclohexanesulfonique.

5. Procédé selon la revendication 1, **caractérisé en ce que** les sels d'acide sulfonique de formule générale VI signifient le p-toluènesulfonate de pyridinium, le p-toluènesulfonate de tétraméthylammonium, le méthanesulfonate de pyridinium et le méthanesulfonate de pyridinium.

6. Procédé selon la revendication 1, **caractérisé en ce que** la préparation des 4,5-allènecétones de formule générale I est réalisée à des températures entre 50°C et 120°C et en travaillant à une pression entre 1 et 20 bars.

7. Procédé selon la revendication 1, **caractérisé en ce que** la préparation des 4,5-allènecétones de formule générale I est réalisée dans un hydrocarbure organique, un aromatique, une cétone ou un éther comme solvant..
